# EUROPEAN PATENT APPLICATION

(11) **EP 3 903 707 A1**
(43) Date of publication of application: **03.11.2021**
(21) Application number: 19902965.3
(22) Date of filing: 20.12.2019
(51) Int. Cl.: A61B 18/12

(54) **DETACHABLE SURGICAL INSTRUMENT**

(30) Priority: 25.12.2018 CN 201811589786
(71) Applicant: Ezisurg Medical Co., Ltd., Shanghai 201203 (CN); Ezisurg (Suzhou) Medical Co., Ltd., Suzhou, Jiangsu 215163 (CN)
(72) Inventor: NIE, Honglin, Shanghai 201318 (CN); YANG, Guang, Shanghai 201318 (CN); CHEN, Jidong, Shanghai 201318 (CN)
(74) Representative: Mansfield, Peter Turquand
(86) International application number: PCT/CN2019/126970
(87) International publication number: WO 2020/135252

(57) **Abstract**

A detachable surgical instrument (10), comprising a distal effector, a blade holder assembly, and a handle assembly (3), the blade holder assembly comprising at least a first blade holder assembly (1) and a second blade holder assembly (2), the first blade holder assembly (1) and the second blade holder assembly (2) being detachably connected to each other, or the second blade holder assembly (2) and the handle assembly (3) being detachably connected to each other, or the first blade holder assembly (1), the second blade holder assembly (2), and the handle assembly (3) being detachably connected to each other. Thus, the problem that common surgical instruments on the market are not easily cleaned after being used and cannot be reused is solved, significantly reducing the usage costs of the instruments, and providing the advantages of a simple structure, convenient assembly and disassembly, and low usage costs.

## Description

### Technical Field

The present disclosure relates to the field of surgical instruments, and in particular, to a detachable surgical instrument.

### Background Art

In the history of the development of surgery, surgical bleeding is one of the important factors that hinder surgical development. Seeking for surgical techniques or methods for cutting and hemostasis is consistent throughout the process of development of surgery, and various kinds of surgical instruments with cutting or hemostasis functions have emerged in succession. Among them, what is the most typical is a high-frequency surgical instrument, which is a surgical instrument for electrocoagulation hemostasis and/or tissue cutting during surgery. The principle of action of the high-frequency surgical instrument is mainly that a blood vessel is clamped and physically pressurized by two clamps at a distal end and meanwhile high-frequency electrical energy is supplied by a high-frequency energy generator, and transmitted to the metal clamps at the distal end of the instrument, so that collagen in the blood vessel is dissolved, denatured, and fused together with fibrin, to close the blood vessel. After the blood vessel is closed, a closed portion may be cut off directly with a cutting blade, thus greatly shortening the operation time, reducing the bleeding, and minimizing the risk of the surgical operation, thus it is widely applied to surgeries due to its high efficiency and reliability with respect to conventional suture ligation.

Existing surgical instruments are mostly disposable instruments, with a shaft thereof being composed of an outer sleeve and an inner sleeve, and a blade holder being located within the inner sleeve. Gaps between the inner and outer sleeves and between the inner sleeve and the blade holder are relatively small. After one surgery is completed, blood or tissue fluid will enter the gap between the inner and outer sleeves or between the inner sleeve and the blade holder. As the gaps are quite small and elongated, it is difficult to clean the blood or tissue fluid that has entered the gaps. Therefore, even if the structure and performance of the surgical instruments are still good, the surgical instruments cannot be reused, and can only be used as disposable instruments, so the cost of using the surgical instruments is quite high.

For the existing reusable high-frequency surgical instruments, only the cutting blade can be used as a disposable component and replaced, but this cannot fundamentally solve the problem that it is difficult to clean the gap between the outer sleeve and the inner sleeve, and there is still the defect of high cost.

### Summary

The present disclosure aims at providing a surgical instrument with a blade holder and a handle assembly being detachable, which has the advantages, compared with the technical solutions that have been disclosed, of a simple structure, convenient assembly and disassembly, easy cleaning, and low usage cost.

In order to achieve the above objectives, the present disclosure provides a detachable surgical instrument, including a distal effector, a blade holder assembly, and a handle assembly (3), wherein the blade holder assembly has a distal end connected to the distal effector, and a proximal end connected to the handle assembly (3), and the blade holder assembly at least includes a first blade holder assembly (1) and a second blade holder assembly (2), wherein the first blade holder assembly (1), the second blade holder assembly (2), and the handle assembly (3) are connected in succession, with at least one of the connections being a detachable connection.

In one aspect, the present disclosure provides a detachable surgical instrument, including a distal effector, a blade holder assembly, and a handle assembly, wherein the blade holder assembly has a distal end connected to the distal effector, and a proximal end connected to the handle assembly, wherein the blade holder assembly at least includes a first blade holder assembly and a second blade holder assembly, and the first blade holder assembly and the second blade holder assembly are detachably connected with each other.

In another aspect, the present disclosure provides a detachable surgical instrument, including a distal effector, a blade holder assembly, and a handle assembly, wherein the blade holder assembly has a distal end connected to the distal effector, and a proximal end connected to the handle assembly, wherein the blade holder assembly at least includes a first blade holder assembly and a second blade holder assembly, and the second blade holder assembly and the handle assembly are detachably connected with each other.

In another aspect, the present disclosure provides a detachable surgical instrument, including a distal effector, a blade holder assembly, and a handle assembly, wherein the blade holder assembly has a distal end connected to the distal effector, and a proximal end connected to the handle assembly, wherein the blade holder assembly at least includes a first blade holder assembly and a second blade holder assembly, and the first blade holder assembly, the second blade holder assembly, and the second blade holder assembly are all detachably connected with each other.

In some optional embodiments, the first blade holder assembly includes a first connecting structure, the second blade holder assembly includes a second connecting structure, and the first blade holder assembly is detachably connected with the second blade holder assembly through the first connecting structure and the second connecting structure.

In some optional embodiments, the first blade holder assembly includes a first outer sleeve, a first inner sleeve, and a first blade holder, the first connecting structure includes the first outer sleeve and the first inner sleeve, the first inner sleeve includes a first metal sleeve and a first insulator, the first insulator is sheathed outside the first metal sleeve, a first metal spring sheet and a second metal spring sheet are mounted on the first insulator, and the first insulator is provided with at least one slot.

In some optional embodiments, the second blade holder assembly includes a second outer sleeve, a second inner sleeve, and a second blade holder, the second connecting structure includes the second outer sleeve and the second inner sleeve, the second inner sleeve includes a second insulator and a second metal sleeve, at least one electrode sheet is provided within the second insulator, and the at least one electrode sheet is connected to the second metal sleeve.

In some optional embodiments, the at least one electrode sheet of the second blade holder assembly is assembled into a corresponding slot of the first insulator of the first blade holder assembly.

In some optional embodiments, the first outer sleeve and the second outer sleeve are connected by threads.

In some optional embodiments, the at least one slot is of a T-shaped structure.

In some optional embodiments, the at least one electrode sheet is of a T-shaped structure.

In some optional embodiments, the second blade holder assembly is detachably connected to the handle assembly.

In some optional embodiments, the second blade holder assembly includes a third connecting structure, the handle assembly includes a fourth connecting structure adapted to the third connecting structure, and the second blade holder assembly is detachably connected to the handle assembly through the third connecting structure and the fourth connecting structure.

In some optional embodiments, the third connecting structure includes a rotation knob, a first disassembly/assembly button assembly, and an outer sleeve connector, and the fourth connecting structure includes a metal connector and a second disassembly/assembly button assembly.

In some optional embodiments, the first disassembly/assembly button assembly includes a first button, a first spring, and a first metal snap ring, wherein after being installed to the first button, the first spring is fixedly connected to the first metal snap ring.

In some optional embodiments, the metal connector includes an annular limiting slot corresponding to the first metal snap ring.

In some optional embodiments, the first disassembly/assembly button assembly can be pressed downwards, so that the first metal snap ring slides downwards, and pushes the second blade holder assembly to be connected with the metal connector along a direction of axis, and after the first metal snap ring enters the annular limiting slot of the metal connector, the first spring enables the first disassembly/assembly button assembly to return to an original position.

Preferably, the surgical instrument in the present disclosure is not limited to an electric knife, an electric hook, a stapler, etc.

Compared with the prior art, the beneficial effects of the present disclosure are as follows:
1) solving the problem that the surgical instruments on the market are not easy to clean after being used and not reusable, and significantly reducing the usage cost of the instruments; and
2) having the advantages of simple structure, convenient assembly and disassembly, and low usage cost.

### Brief Description of Drawings

Accompanying drawings, constituting a portion of the description, are used to provide further understanding to the present disclosure, and explain the present disclosure together with the specific embodiments of the present disclosure, rather than limiting the present disclosure.
FIG. 1 is a schematic view of a detachable surgical system;
FIG. 2 is a schematic view of a detachable surgical instrument;
FIG. 3 is a schematic view of the detachable surgical instrument being disassembled;
FIG. 4 is a structural composition diagram of a first blade holder assembly, with a lower right corner schematically showing an enlarged view of a first connecting structure;
FIG. 5 is a structural composition diagram of a second blade holder assembly, with a lower left corner schematically showing an enlarged view of a second connecting structure;
FIG. 6 is a schematic view of the first blade holder assembly and the second blade holder assembly being pre-assembled;
FIG. 7 is a schematic view of the first blade holder assembly and the second blade holder assembly being assembled in place;
FIG. 8 is a schematic view of the blade holder assemblies and a handle being pre-assembled;
FIG. 9 is a schematic view of the blade holder assemblies and the handle assembly being assembled in place, with an enlarged view of assembling a third connecting structure and a fourth connecting structure schematically shown below; and
FIG. 10 is a schematic view of the blade holder assemblies and the handle assembly being assembled.

### Detailed Description of Embodiments

The present disclosure is further described below in combination with specific embodiments, wherein the drawings are merely for exemplary description, merely represent schematic views, but not physical diagrams, and cannot be construed as limitation to the present invention. In order to better illustrate specific embodiments of the present disclosure, some components in the drawings are omitted, enlarged or scaled down, but do not represent the size of an actual product. For a person skilled in the art, omission of certain well-known structures in the drawings and the description thereof may be understood. Based on the specific embodiments in the present disclosure, all of other specific embodiments obtained by a person of ordinary skill in the art without making creative efforts belong to the scope of protection of the present disclosure.

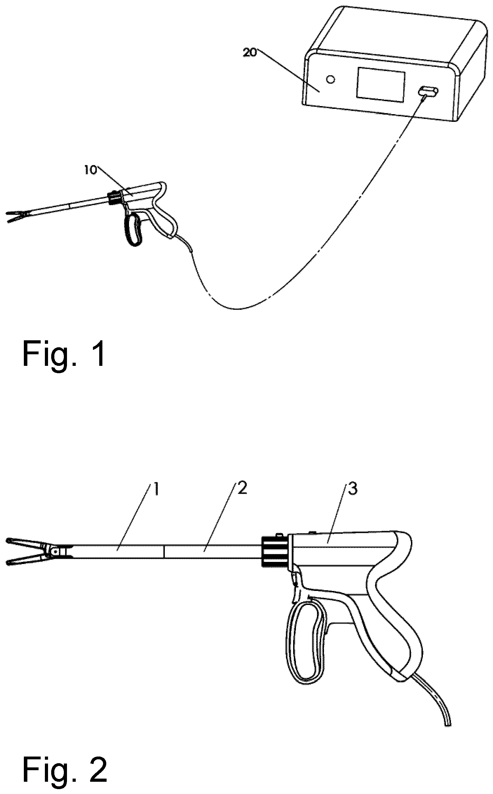

A surgical instrument system, as shown in FIG. 1, is mainly composed of a detachable electrosurgical instrument 10 and a high-frequency current generator 20. Therein, the high-frequency current generator 20 generates high-frequency electric energy which is transmitted to a metal clamp at a distal end of the high-frequency electrosurgical instrument, to close (coagulate or seal) a blood vessel.

In an embodiment, as shown in FIG. 2, the detachable electrosurgical instrument 10 includes a distal effector, a first blade holder assembly 1, a second blade holder assembly 2, and a handle assembly 3.

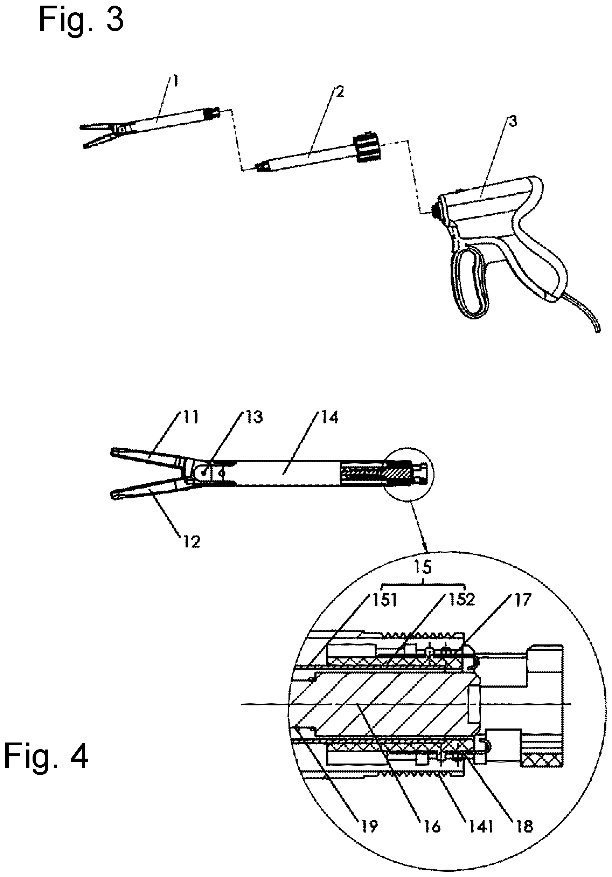

As shown in FIG. 3, the first blade holder assembly 1, the second blade holder assembly 2, and the handle assembly 3 may achieve convenient installation and disassembly.

As shown in FIG. 4, the first blade holder assembly 1 includes a first outer sleeve 14, a first inner sleeve 15, and a first blade holder 16, wherein a distal end of the first blade holder assembly 1 is connected to the distal effector, and a proximal end includes a first connecting structure.

In an embodiment, as shown in FIG. 4, the distal effector includes a first clamp 11, a second clamp 12, and a fixed pin shaft 13; the first clamp 11 and the second clamp 12 are fixedly connected to the first outer sleeve 14 through the fixed pin shaft 13, wherein the fixed connection may be realized in a variety of manners such as welding, riveting or threaded connection, and the two clamps may rotate about the fixed pin shaft 13, thereby realizing closure and opening between the clamps. In other embodiments, the distal effector is not limited to a pair of clamp structures, for example, forceps, etc. The distal effector may be manipulated in a manner of manipulating the effector in the surgical instruments in the prior art.

The detailed structure of the first connecting structure of the first blade holder assembly 1 is as shown in FIG. 4. The first connecting structure includes a first outer sleeve 14 and a first inner sleeve 15. In the present embodiment, the first inner sleeve 15 includes a first metal sleeve 151 and a first insulator 152 sheathed outside the first metal sleeve 151, and the fixed connection thereof may be realized in a variety of manners such as co-injection, gluing or welding. The first inner sleeve 15 further includes a first metal spring sheet 17 and a second metal spring sheet 18 mounted on the first insulator 152, functioning to ensure the reliability of electrical connection between the first blade holder assembly 1 and the second blade holder assembly 2, and the fixed connection thereof may be realized in a variety of manners such as hot melting, co-injection or gluing.

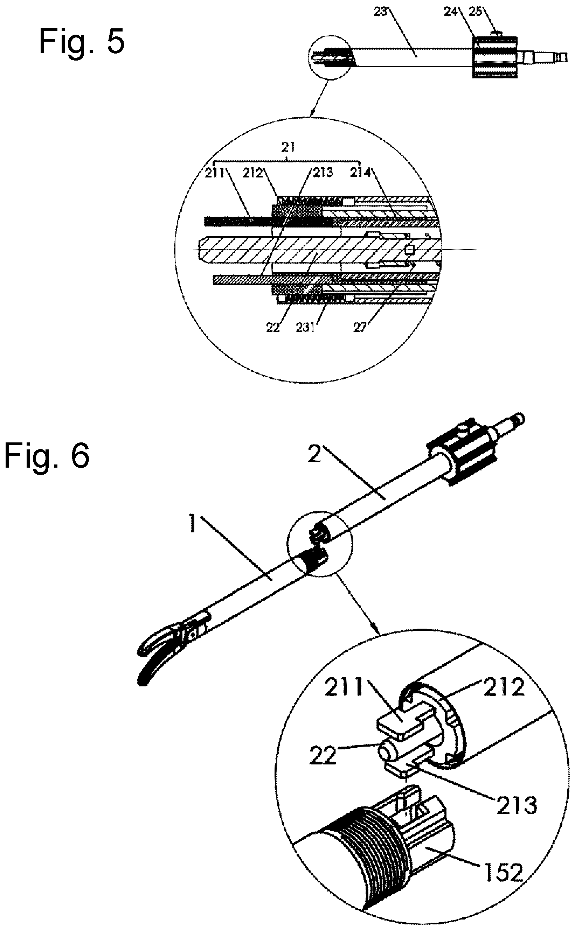

In an embodiment, as shown in FIG. 6, the first insulator 152 is provided with a pair of slots. In an embodiment, the slots are T-shaped, but are not limited thereto.

As shown in FIG. 5, the second blade holder assembly 2 includes a second outer sleeve 23, a second inner sleeve 21, and a second blade holder 22. A distal end of the second blade holder assembly 2 includes a second connecting structure that is adapted to the first connecting structure. A proximal end of the second blade holder assembly 2 includes a third connecting structure that is adapted to the fourth connecting structure of the handle assembly 3.

The detailed structure of the second connecting structure of the second blade holder assembly 2 is as shown in FIG. 5, and the second connecting structure includes the second inner sleeve 21 and the second outer sleeve 23. In an embodiment, the second inner sleeve 21 includes a first electrode sheet 211, a second electrode sheet 213, a second insulator 212, and a second metal sleeve 214. The second insulator 212 is sheathed outside the first electrode sheet 211 and the second electrode sheet 213. The fixed connection of the second insulator 212 may be realized in a variety of manners, such as co-injection, gluing, or welding.

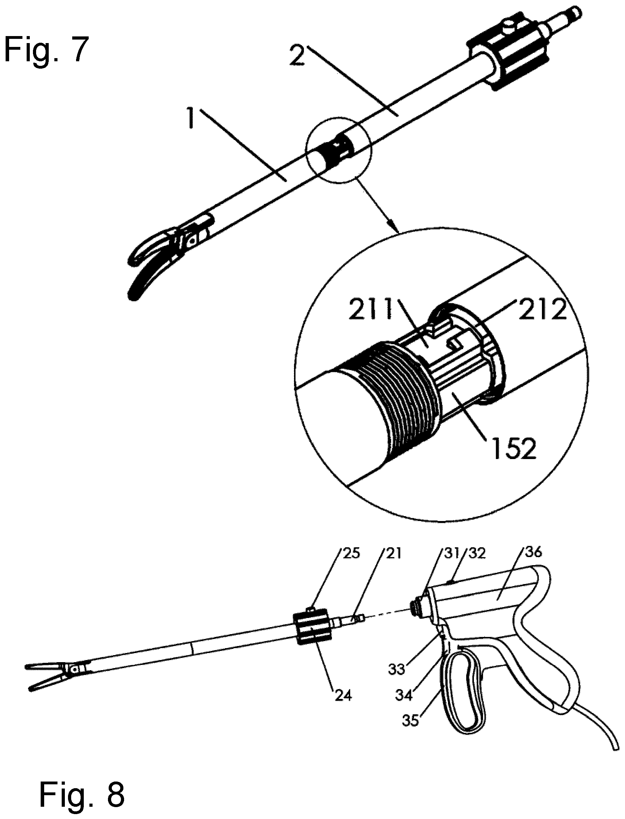

As shown in FIG. 6 and FIG. 7, the function of installing or disassembling the first blade holder assembly 1 and the second blade holder assembly 2 is mainly realized by connecting/disassembling the first inner sleeve 15 and the second inner sleeve 21, and connecting/disassembling the first outer sleeve 14 and the second outer sleeve 23. The first electrode sheet 211 and the second electrode sheet 213 of the second blade holder assembly 2 which are of a T-shaped structure are installed, along a direction perpendicular to axes of the blade holders, in the T-shaped slots corresponding thereto in the first insulator 152 of the first blade holder assembly 1, until being in contact with a limit bottom surface, at which time, the first metal spring sheet 17 and the first electrode sheet 211 are in contact with each other, and the second metal spring sheet 18 and the second electrode sheet 213 are in contact with each other; the first blade holder 16 and the second blade holder 22 are kept in elastic contact by a pre-pressure applied by a spring 19 (see FIG. 4) of the first blade holder assembly 1 and a spring 27 (see FIG. 5) of the second blade holder assembly 2; after completing the above steps, the first outer sleeve 14 and the second outer sleeve 23 are fixedly connected together by a thread 141 (see FIG. 4) and a thread 231 (see FIG. 5), to form a complete blade holder assembly.

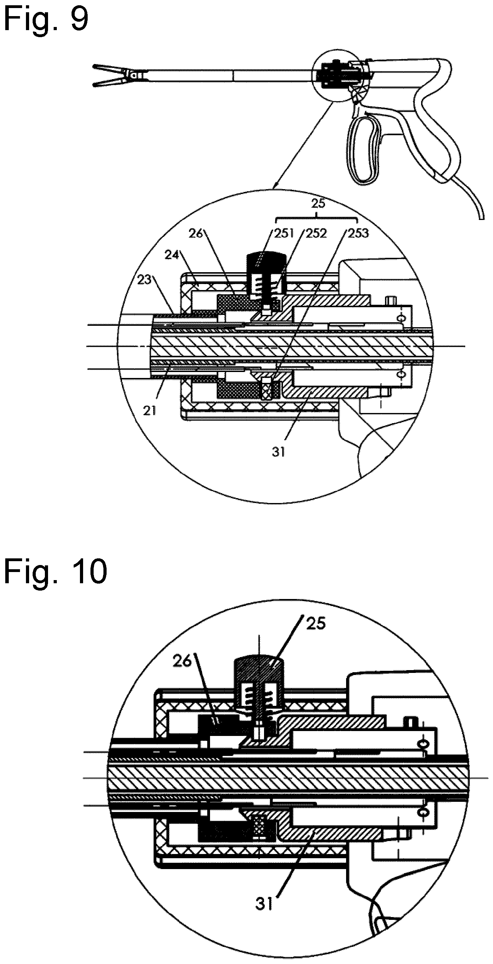

As shown in FIG. 8 and FIG. 9, the proximal end of the second blade holder assembly 2 includes a third connecting structure, and a distal end of the handle assembly 3 includes a fourth connecting structure adapted to the third connecting structure. The third connecting structure includes a rotation knob 24, a first disassembly/assembly button assembly 25, and an outer sleeve connector 26.

The handle assembly 3 includes a metal connector 31, a second disassembly/assembly button assembly 32, a power button 33, a cutting trigger 34, a clamping trigger 35, and a handle body 36.

The function of installing or disassembling the second blade holder assembly 2 and the handle assembly 3 is mainly realized by connecting or disassembling the first disassembly/assembly button assembly 25, the outer sleeve connector 26, and the metal connector 31, and connecting or disassembling the second disassembly/assembly button assembly 32 and the second inner sleeve 21.

The connecting structure of the first disassembly/assembly button assembly 25 and the metal connecting member 31 is as shown in FIG. 9. The second outer sleeve 23 and the outer sleeve connector 26 are fixedly connected with each other by a variety of manners such as co-injection, welding, threaded connection or gluing, etc., and the first disassembly/assembly button assembly 25 includes a first button 251, a first spring 252, and a first metal snap ring 253. After being installed to the first button 251, the first spring 252 is fixedly connected to the first metal snap ring 253, wherein there are a variety of fixed connection manners such as threaded connection, crimping or gluing. After the fixed connection, the first spring 252 has a pre-pressure on the outer sleeve connector 26, so that the first metal snap ring 253 cannot cooperate with an annular limiting slot of the metal connector 31 in a natural state.

Detailed installation structures and steps of the second blade holder assembly 2 and the handle assembly 3 are as shown in FIG. 8, FIG. 9, and FIG. 10. The first disassembly/assembly button assembly 25 is pressed in a direction perpendicular to the axes of the blade holders, the first metal snap ring 253 slides downwards, and pushes the second blade holder assembly 2 to be installed with the metal connector 31 of the handle in the direction of the axes of the blade holders. After the first metal snap ring 253 enters the annular limiting slot of the metal connector 31, the first disassembly/assembly button assembly 25 returns to an original position under the action of the first spring 252, thus realizing the connection and fixation of the second blade holder assembly 2 and the handle assembly 3. In a similar fashion, the same structure and fixed connection manner may be applied to the connecting structure of the second disassembly/assembly button assembly 32 and the second inner sleeve 21, thus realizing the opening or closing action of the first clamp 11 and the second clamp 12.

It should be noted that the embodiments in the drawings are merely representative embodiments of the present disclosure, the scope of protection of the present disclosure is not merely limited to the scope defined by the embodiments in the drawings, and combinations, modifications, and changes to the embodiments in the drawings all belong to the scope of protection of the present disclosure. Although specific embodiments of the present disclosure have been shown and described, for those ordinarily skilled in the art, it could be understood that various changes, amendments, substitutions, and modifications could be made to these specific embodiments without departing from the principle and spirit of the present disclosure, and the scope of the present disclosure is defined by the appended claims and their equivalents.

## Claims

1. A detachable surgical instrument, comprising a distal effector, a blade holder assembly, and a handle assembly (3), the blade holder assembly having a distal end connected to the distal effector and a proximal end connected to the handle assembly (3), wherein the blade holder assembly at least comprises a first blade holder assembly (1) and a second blade holder assembly (2), wherein the first blade holder assembly (1), the second blade holder assembly (2), and the handle assembly (3) are connected in succession, with at least one connection being a detachable connection.

2. The detachable surgical instrument according to claim 1, wherein the first blade holder assembly (1) comprises a first connecting structure, the second blade holder assembly (2) comprises a second connecting structure, and the first blade holder assembly (1) is detachably connected with the second blade holder assembly (2) through the first connecting structure and the second connecting structure.

3. The detachable surgical instrument according to claim 1, wherein the second blade holder assembly (2) comprises a third connecting structure, the handle assembly (3) comprises a fourth connecting structure adapted to the third connecting structure, and the second blade holder assembly (2) is detachably connected with the handle assembly (3) through the third connecting structure and the fourth connecting structure.

4. The detachable surgical instrument according to claim 2, wherein the second blade holder assembly (2) comprises a third connecting structure, the handle assembly (3) comprises a fourth connecting structure adapted to the third connecting structure, and the second blade holder assembly (2) is detachably connected with the handle assembly (3) through the third connecting structure and the fourth connecting structure.

5. The detachable surgical instrument according to claim 2 or 4, wherein the first blade holder assembly (1) comprises a first outer sleeve (14), a first inner sleeve (15), and a first blade holder (16), the first connecting structure comprises a first outer sleeve (14) and a first inner sleeve (15), wherein the first inner sleeve (15) comprises a first metal sleeve (151) and a first insulator (152) sheathed outside the first metal sleeve (151), a first metal spring sheet (17) and a second metal spring sheet (18) are mounted on the first insulator (152), and the first insulator (152) is provided with at least one slot.

6. The detachable surgical instrument according to claim 5, wherein the at least one slot is of a T-shaped structure.

7. The detachable surgical instrument according to claim 2 or 4, wherein the second blade holder assembly (2) comprises a second outer sleeve (23), a second inner sleeve (21), and a second blade holder (22), the second connecting structure comprises a second outer sleeve (23) and a second inner sleeve (21), the second inner sleeve (21) comprises a second insulator (212) and a second metal sleeve (214), at least one electrode sheet is provided within the second insulator (212), and the at least one electrode sheet is connected to the second metal sleeve (214).

8. The detachable surgical instrument according to claim 7, wherein the at least one electrode sheet is of a T-shaped structure.

9. The detachable surgical instrument according to claim 5, wherein the second blade holder assembly (2) comprises a second outer sleeve (23), a second inner sleeve (21), and a second blade holder (22), the second connecting structure comprises a second outer sleeve (23) and a second inner sleeve (21), the second inner sleeve (21) comprises a second insulator (212) and a second metal sleeve (214), at least one electrode sheet is provided within the second insulator (212), and the at least one electrode sheet is connected to the second metal sleeve (214).

10. The detachable surgical instrument according to claim 9, wherein the at least one electrode sheet is of a T-shaped structure.

11. The detachable surgical instrument according to claim 10, wherein the at least one electrode sheet of the second blade holder assembly (2) is assembled into a corresponding slot of the first insulator (152) of the first blade holder assembly (1).

12. The detachable surgical instrument according to claim 9, wherein the first outer sleeve (14) and the second outer sleeve (23) are connected by threads (141, 231).

13. The detachable surgical instrument according to claim 3 or 4, wherein the third connecting structure comprises a rotation knob (24), a first disassembly/assembly button assembly (25), and an outer sleeve connector (26), and the fourth connecting structure comprises a metal connector (31) and a second disassembly/assembly button assembly (32).

14. The detachable surgical instrument according to claim 13, wherein the first disassembly/assembly button assembly (25) comprises a first button (251), a first spring (252), and a first metal snap ring (253), wherein the first spring (252) is configured to be fixedly connected to the first metal snap ring (253) after being installed to the first button (251).

15. The detachable surgical instrument according to claim 13, wherein the metal connector (31) comprises an annular limiting slot corresponding to the first metal snap ring (253).

16. The detachable surgical instrument according to claim 15, wherein the first disassembly/assembly button assembly (25) is configured to be able to be pressed downwards, to make the first metal snap ring (253) slide downwards and push the second blade holder assembly (2) to be connected with the metal connector (31) along a direction of axis, and the first spring (252) is configured to make the first disassembly/assembly button assembly (25) to return to an original position after the first metal snap ring (253) enters the annular limiting slot of the metal connector (31).

17. The detachable surgical instrument according to claim 1, wherein the surgical instrument is an electric knife, an electric hook or a stapler.
